# EUROPEAN PATENT SPECIFICATION

(11) **EP 1 357 888 B1**
(45) Date of publication and mention of the grant of the patent: **16.09.2009**
(21) Application number: 02729559.1
(22) Date of filing: 11.01.2002
(51) Int. Cl.: A61K 8/37

(54) **HAIR TREATMENT AGENT**
MITTEL ZUR BEHANDLUNG DES HAARES
AGENT DE TRAITEMENT CAPILLAIRE

(30) Priority: 15.01.2001 JP 2001006457; 21.02.2001 US 269853 P
(43) Date of publication of application: 05.11.2003
(73) Proprietor: Showa Denko K.K., Tokyo 105-8518 (JP)
(72) Inventor: ITO, Naoko, c/o SHOWA DENKO K.K., Ct. Res. Lab., Chiba-shi, Chiba 267-0056 (JP); KATO, Eiko, c/o SHOWA DENKO K.K., Ct. Res. Lab., Chiba-shi, Chiba 267-0056 (JP); TSUZUKI, Toshi, c/o SHOWA DENKO K.K., Ct. Res.Lab., Chiba-shi, Chiba 267-0056 (JP)
(74) Representative: Strehl Schübel-Hopf & Partner
(86) International application number: PCT/JP2002/000149
(87) International publication number: WO 2002/055041

(56) References cited:
- DATABASE CA [Online] CHEMICAL ABSTRACTS SERVICE, COLUMBUS, OHIO, US; ITO SHINOBU: "Hair quality improving preparations containing radical scavengers for veterinary use" Database accession no. 136:172475 XP002215863 & JP 2002 047182 A 12 February 2002 (2002-02-12)
- DATABASE CA [Online] CHEMICAL ABSTRACTS SERVICE, COLUMBUS, OHIO, US; IKENO HIROSHI: "Radial-scavenging topical compositions" Database accession no. 134:285477 XP002215864 & JP 2001 097888 A 10 April 2001 (2001-04-10)
- DATABASE CA [Online] CHEMICAL ABSTRACTS SERVICE, COLUMBUS, OHIO, US; ITO, SHINOBU: "Highly stable ascorbic acid derivative compositions" Database accession no. 134:61235 XP002215865 & JP 2000 351905 A 19 December 2000 (2000-12-19)
- DATABASE CA [Online] CHEMICAL ABSTRACTS SERVICE, COLUMBUS, OHIO, US; SAKAMOTO,TETSUE ET AL.: "Hair preparations containing glycosylascorbic acid" Database accession no. 118:11500 XP002215866 & JP 04 182414 A 30 June 1992 (1992-06-30)
- PATENT ABSTRACTS OF JAPAN vol. 2000, no. 20, 10 July 2001 (2001-07-10) & JP 2001 079568 A (SHOWA DENKO KK), 27 March 2001 (2001-03-27)

## Description

### DETAILED DESCRIPTION OF THE INVENTION

### Application Field in Industry

The present invention relates to a hair treatment agent, a hair care composition and uses thereof, more specifically, the present invention relates to a hair treatment agent, a hair care composition and uses thereof, which can prevent hair damages ascribable to chlorine compounds added to tap water or water in a swimming pool for the purpose of sterilization or free chlorine generated from these chlorine compounds and which can also prevent hair damages ascribable to hydroxy radicals originated in various radical generators present in ultraviolet rays or environment.

### Technical Background of the Invention

Conventionally, hair damages ascribable to chlorine compounds added to tap water or water in a swimming pool for the purpose of sterilization or free chlorine generated from these chlorine compounds have been pointed out. Upon exposure to free chlorine, hair damages such as decolorization, reduction in the strength or decrease in the moisture retentivity of hair, so-called generation of creaky feeling or loss of smoothness, and increase in split ends or falling hair may occur. These phenomena are considered to result from oxidative decolorization of melanin in the hair structure by free chlorine, oxidative denaturing of protein covering the hair surface and disintegration of cuticle structure ascribable to these oxidative actions.

In recent years, some hair treatment agents have been proposed so as to prevent these damages. For example, JP-A-3-153616 (the term "JP-A" as used herein means an "unexamined published Japanese patent application") discloses a remover for chlorine attached hair, comprising methionine, proline or tryptophan of amino acid, and a hair treatment composition containing the remover. According to the knowledge of the present inventors, however, these amino acid ingredients are not necessarily satisfied regarding dechlorination rate and removal ratio. Furthermore, these amino acids lack in the hydroxy radical-scavenging ability which is described later, and therefore, cannot prevent hair damage caused by hydroxy radicals.

Also, for example, U.S. Patent 4,295,985 discloses a soap or a shampoo containing sodium thiosulfate as a dechlorination ingredient. Despite high dechlorinating ability, sodium sulfate has a problem of, when used in excess, seriously damaging hair caused by its strong reducing property.

In addition to this hair damage ascribable to chlorine, hair or skin damages caused by radicals generated under the action of an ultraviolet ray are recently coming to a problem. In particular, hydroxyl radical is known to be highly reactive and oxidative-decompose lipid, protein or melanin, thereby seriously damaging the hair or skin.

In order to solve this problem, many radical scavengers have been proposed mainly for the purpose of skin protection. Examples of this radical scavenger include polyphenols. However, the polyphenols give high irritation to skin, though its radical-scavenging ability is high. Also, hair protective agents having radical-scavenging ability have been proposed, which, however, cannot be evaded from contacting with skin of head or face on administration or use. Thus, the problem of irritation on application to skin still remains.

JP-A-62-126112 discloses a hair treatment composition containing an ascorbic acid as an ingredient having antioxidant property. The ascorbic acid is originally a biological ingredient and therefore, is safe. Moreover, the ascorbic acid has excellent properties as an ingredient of hair treatment agents, such as high dechlorinating and hydroxy radical-scavenging abilities and relatively low irritation to skin. However, the ascorbic acid is deficient in that the stability is extremely low at ordinary temperatures and in the presence of oxygen and the effects thereof are lost within a short period of time.

WO 95/17157 discloses the use of hair preparations comprising L-ascorbic acid (Vit.C) for the treatment of human hair against environmental influences, esp. for the pre- or post-treatment following irradiation with light and/or lasers. L-ascorbic acid has a known radical-scavenging and antioxidant activity (see page 1 lines 17-22, examples and claim 9).

### Mode for Carrying Out the Invention

Under these circumstances, the present inventors have made extensive investigations to solve the above-described problems, as a result, it has been found that some kinds of ascorbic acid derivatives exhibit high dechlorinating and hydroxy radical-scavenging abilities, excellent safety to skin, low irritation and sufficiently high storage stability required in view of the production of preparations, and therefore, these derivatives are an excellent effective ingredient for hair treatment agents. The present invention has been accomplished based on this finding.

### Object of the Invention

An object of the present invention is to solve the above-described problems and provide a hair treatment, a hair care composition and uses thereof, ensuring hydroxy radical-scavenging abilities, excellent safety to skin, low irritation and sufficiently high storage stability required in view of the production of preparations.

### Summary of the Invention

A use of a composition of a hair treatment agent to prevent damage of the hair caused by hydroxyl radicals of the present invention is characterized by comprising, as an effective ingredient, an ascorbic acid derivative which is a compound represented by formula (1) or a salt thereof: 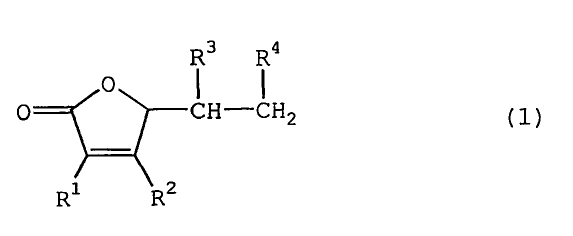 (wherein R¹, R², R³ and R⁴ each independently represents a hydroxyl group, an ester bond-containing group derived from a hydroxyl group and an organic group or an inorganic group, or R¹ and R² or R³ and R⁴ may form together a divalent group derived from two adjacent hydroxyl groups and aldehyde or ketone, provided that R¹ and R² are not a hydroxyl group at the same time).

The above-described ascorbic acid derivative preferably has a hydroxyl radical-scavenging ability.

The above-described ester bond-containing group is preferably an acyloxy group or a group selected from the group consisting of a sulfuric acid group, a phosphoric acid group, a pyrophosphoric acid group, a triphosphoric acid group and polyphosphoric acid group.

The above-described ascorbic acid derivative is preferably ascorbyl 2-phosphate ester or a salt thereof, or ascorbyl 2-glucoside or a derivative thereof.

In the case where the above-described hair treatment agent is in a liquid form, the ascorbic acid derivative content in the hair treatment agent is preferably from 0.01 to 50% by mass.

The hair care composition of the present invention is characterized by comprising the above-described hair treatment agent.

The hair care composition can be preferably used for hair-styling and also for pets.

This hair care composition is preferably in the form of shampoo, pet shampoo, rinse, hair conditioner, hair tonic or styling jell.

### Detailed Description of the Invention

The hair treatment agent of the present invention is described in detail below.

The hair treatment agent according to the present invention comprises an ascorbic acid derivative as an effective ingredient. The ascorbic acid derivative is a compound represented by the following formula (1) or a salt thereof.

### Ascorbic Acid Derivative

The ascorbic acid derivative for use in the present invention is a compound represented by formula (1) or a salt thereof: 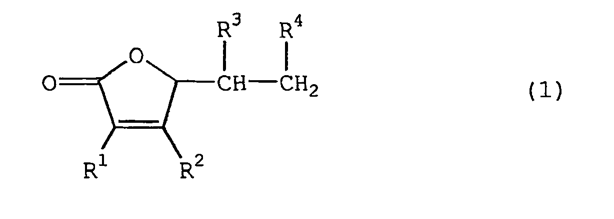 (wherein R¹, R², R³ and R⁴ each independently represents a hydroxyl group, an ester bond-containing group derived from a hydroxyl group and an organic group or an inorganic group, or R¹ and R² or R³ and R⁴ may form together a divalent group derived from two adjacent hydroxyl groups and aldehyde or ketone, provided that R¹ and R² are not a hydroxyl group at the same time).

In the present invention, this ascorbic acid derivative preferably has a hydroxy radical-scavenging ability. The term "a hydroxy radical-scavenging ability" means an ability of swiftly reacting with a hydroxy radical to reduce the hydroxy radical into a low reactive compound or a low reactive ion species.

In the present invention, the ascorbic acid derivative is preferably a compound represented by formula (1) or a salt thereof, wherein R¹, R², R³ and R⁴ each independently represents a hydroxyl group, an ester bond-containing group derived from a hydroxyl group and an organic group or an inorganic group, or R¹ and R² or R³ and R⁴ may form together a divalent group derived from two adjacent hydroxyl groups and aldehyde or ketone. In formula (1), R¹ and R² are not a hydroxyl group at the same time. As such, in formula (1), R¹ and R² are not a hydroxyl group at the same time and therefore, the compound or a salt derived therefrom is considered not prone to oxidization and capable of exhibiting excellent safety.

In other words, the ascorbic acid derivative for use in the present invention is a compound or a salt thereof, where a part of hydroxyl groups at 2-, 3-, 5- and 6-positions of an ascorbic acid each is independently esterified by an inorganic or organic acid or glycosylated by a sugar; or the adjacent hydroxyl groups at 2- and 3- or 5- and 6- positions are acetalized or ketalized to form a divalent group (provided that 2- and 3-positions are not a hydroxyl group at the same time) and where R¹, R², R³ and R⁴ may be different from each other.

Examples of such an ascorbic acid derivative include organic acid esters of ascorbic acid obtained by the esterification reaction of an ascorbic acid with an organic acid, inorganic acid esters of ascorbic acid obtained by the esterification reaction of an ascorbic acid with an inorganic acid, glycosides of ascorbic acid obtained by the dehydration condensation of an ascorbic acid with a sugar, ketal forms obtained by the reaction of two adjacent hydroxyl groups in an ascorbic acid with ketone, and acetal forms obtained by the reaction of two adjacent hydroxyl groups in an ascorbic acid with aldehyde.

The ascorbic acid used in the formation of an ascorbic acid derivative for use in the present invention may be any one of D-isomer, L-isomer and DL-isomer, however, L-isomer is preferred in view of the relation to natural vitamin C.

Examples of the organic acid used for the esterification of ascorbic acid include carboxylic acids represented by the formula: RCOOH (wherein R is a hydrocarbon group having from 1 to 30, preferably from 1 to 17, carbon atoms, which may be saturated or unsaturated, may be linear or branched and may have an aromatic group), and sulfonic acids represented by the formula: RSO₃H (wherein R has the same meaning as above).

Specifically, the carboxylic acid (RCOOH) is preferably a fatty acid having from 1 to 30 carbon atoms and examples thereof include acetic acid, propionic acid, butyric acid, isobutyric acid, stearic acid, myristic acid and palmitic acid. Among these, preferred are fatty acids having from 1 to 17 carbon atoms, more preferred are higher fatty acids having from 13 to 17 carbon atoms, and still more preferred is palmitic acid.

Examples of the sulfonic acid (RSO₃H) include p-toluene sulfonic acid.

Specific examples of the ester bond-containing group derived from a hydroxyl group and the above-described organic acid include acyloxy groups such as acetoxy group, propionyloxy group, butyryloxy group, isobutyryloxy group, stearoyloxy group, myristoyloxy group, palmitoyloxy group and lauroyloxy group. Among these, palmitoyloxy group is preferred.

Examples of the inorganic acid for use in the esterification of ascorbic acid include phosphoric acid, pyrophosphoric acid, triphosphoric acid, tetraphosphoric acid, polyphosphoric acid and sulfuric acid. Among these, phosphoric acid is preferred.

Specific example of the ester-bond group derived from a hydroxyl group and the above-described inorganic acid include a phosphoric acid group (-OPO₃H₂), a pyrophosphoric acid group (-OP₂O₆H₃), a triphosphoric acid group (-OP₃O₉H₄), a sulfuric acid group (-OSO₃H) and polyphosphoric acid group. Among these, a phosphoric acid group (-OPO₃H₂) is preferred.

The sugar for use in the glycosylation of ascorbic acid may be either monosaccharide or polysaccharide and specific examples thereof include monosaccharides such as glucose, fructose, galactose and mannose, oligosaccharides such as sucrose, lactose and maltose, and polysaccharides such as cellulose, starch and glycogen. Among these, glucose is particularly preferred. In the present invention, a hydroxyl group of the sugar may be substituted.

The glycoside group derived from such a sugar may be originated in either a monosaccharide or a polysaccharide and examples of the glycoside group include a glucoside group, a fructoside group, a galactoside group and a mannoside group. Among these, a glucoside group is particularly preferred.

In formula (1), R¹ and R² or R³ and R⁴ may form together a divalent group derived from two adjacent hydroxyl groups of an ascorbic acid, and aldehyde or ketone.

Examples of such a group include the groups represented by the following formula (2a): 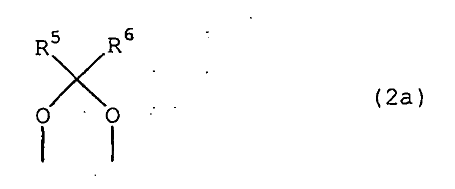 (wherein R⁵ and R⁶ each independently represents hydrogen or a hydrocarbon group).

In the case where R⁵ and R⁶ in formula (2a) are a hydrocarbon group, the hydrocarbon group may be aliphatic, alicyclic or aromatic, may be linear or branched, and may be saturated or unsaturated. In the case where the hydrocarbon group is an aliphatic hydrocarbon group, a linear aliphatic hydrocarbon is preferred and the number of carbon atoms thereof is preferably from 1 to 10, more preferably from 1 to 5. Specific examples of such an aliphatic hydrocarbon group include a methyl group, an ethyl group, a propyl group and a butyl group. Specific examples of the alicyclic hydrocarbon group include a cyclohexyl group, and specific examples of the aromatic hydrocarbon group include a phenyl group and a benzyl group.

In the case where R³ and R⁴ in formula (1) are a group represented by formula (2a), the ascorbic acid derivative forms, for example, an acetal or ketal structure represented by the following formula (2):

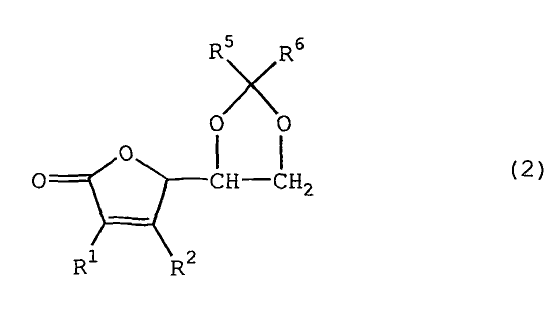

Examples of the ketone used in the reaction with the ascorbic acid to form a ketal form (structure) include acetone, ethyl methyl ketone and diethyl ketone.

Examples of the aldehyde used in the reaction with the ascorbic acid to form an acetal form (structure) include acetaldehyde, propionaldehyde and benzaldehyde.

The ascorbic acid derivative may be a salt of the compound represented by formula (1) and examples of the salt include metal salts and ammonium salts.

In the case where the ascorbic acid derivative is a metal salt of the compound represented by formula (1), examples of the metal used for forming a salt include alkali metals such as sodium and potassium, alkaline earth metals such as magnesium and calcium, and zinc. Among these, sodium and magnesium are preferred.

In the case where the ascorbic acid derivative is an ammonium salt of the compound represented by formula (1), the ammonium salt may be an alkyl group-substituted ammonium salt or a hydroxyalkyl group-substituted ammonium salt.

Examples of such an ascorbic acid derivative include ascorbyl 2-phosphate, ascorbyl 2-diphosphate, ascorbyl 2-triphosphate, ascorbyl 2-polyphosphate, ascorbyl 2,6-phosphate diester, ascorbyl 2-phosphate-6-palmitate, ascorbyl 2-phosphate-6-myristate, ascorbyl 2-phosphate -6-stearate, ascorbyl 2-phosphate-6-oleate, ascorbyl 2-glucoside, ascorbyl 2-glucoside-6-palmitate, ascorbyl 2-glucoside-6-myristate, ascorbyl 2-glucoside-6-stearate, ascorbyl 2-glucoside-6-oleate, ascorbyl 2-sulfate, and their sodium salt, potassium salt, magnesium salt, calcium salt, zinc salt, ammonium salt, alkyl-substituted ammonium salt and hydroxyalkyl-substituted ammonium salt.

In the present invention, as long as the ascorbic acid derivative has hydroxy radical-scavenging abilities, the ascorbic acid derivative may be introduced into an arbitrary polymer chain as a side chain or a unit of the ascorbic acid derivative may be integrated into the polymer main chain.

These ascorbic acid derivatives can be produced by appropriately combining conventionally known methods.

### Production of Ascorbic Acid Derivatives

The method for producing ascorbic acid derivatives is described in detail below by referring to preferred examples thereof, however, the method for producing ascorbic acid derivatives is not limited to these specific examples.

For example, the above-described ascorbyl 2-phosphate ester can be produced by dissolving an ascorbic acid in a mixed solvent of pyridine and water, and reacting it with a phosphorylating agent such as phosphorus oxychloride while adjusting the pH to from 10 to 13 by an aqueous solution of an alkali metal hydroxide such as sodium hydroxide and potassium hydroxide.

The alkali metal salt of ascorbyl 2-phosphate ester, for example, ascorbyl 2-phosphate ester magnesium salt can be produced by a general method such as a method of reacting an ascorbyl 2-phosphate ester with a magnesium compound (e.g., magnesium oxide, magnesium hydroxide).

### Hair Treatment Agent

The hair treatment agent according to the present invention may have any preparation form as long as the hair treatment contains the above-described ascorbic acid derivative, for example, a solid solution form such as jelly, a solid form such as tablet, powder and granule, or a liquid form.

Among those ascorbic acid derivatives, in the case where the hair treatment agent is in the liquid form, ascorbyl 2-phosphate ester, ascorbyl 2-glucoside and their salts with the above-described metals are preferred in view of convenience in the production of preparations (for example, water solubility), chemical stability of the derivative blended in products, and effects.

Depending upon the preparation shape, the hair treatment agent may be used, if desired, by dissolving it in purified water, lotion or hair oil (e.g., tsubaki oil, squalane oil) or may be used as a composition imparted with other functions such as rinsing, moisture retention, nutrient supply, ultraviolet absorption and physical protection, by mixing it with other ingredients.

The hair treatment agent of the present invention comprises an ascorbic acid derivative as an effective ingredient which is derived from natural material and therefore, this agent is not only excellent in the safety to skin, low in the irritation, and safe and harmless even if the ingredient penetrates into a subcutaneous tissue through skin of a human being or an animal other than human being, but also advantageous in that, by the subcutaneous penetration, the synthesis of collagen in the skin tissue is stimulated and the activity of protecting the skin tissue from external factors such as ultraviolet ray is strengthened. Accordingly, the hair treatment agent of the present invention can be suitably used for protecting hair of human beings. In addition, since the ascorbic acid derivatives are odorless, the hair treatment agent of the present invention can also be suitably used for hair of pets which are highly sensitive to odors.

In the case where the hair treatment agent is in the liquid form, the amount blended of the ascorbic acid derivative contained in the hair treatment agent of the present invention is preferably from 0.01 to 50% by mass, more preferably from 0.1 to 20% by mass, based on 100% by mass as a total of the hair treatment agent. When the ascorbic acid derivative is contained in the hair treatment agent in such an amount, the effect obtained thereof can be sufficiently high to eliminate chlorine contained in ordinary tap water and moreover, the solvent used for preparing the ascorbic acid derivative can be selected over a wide range without any limitation on the kind.

### Blended Composition of Hair Treatment Agent

In addition to the above-described ascorbic acid derivative as the essential ingredient, the hair treatment agent usually contains a base and furthermore, "other ingredients" generally used as cosmetic ingredients are appropriately blended, if desired. In the hair treatment agent of the present invention, for example, the ingredients described in the Japanese Standards of Cosmetic Ingredients (JSCI), "Specifications of Ingredients Other Than Those Listed in JSCI" (1993 supplement, issued by Yakuji Nippo, Ltd.) can be blended.

The base for the hair treatment agent may be any base as long as it is generally used as a cosmetic ingredient, and examples thereof include purified water, mineral water, ethanol, glycerin, squalane, 1,3-propylene glycol, 1,3-butylene glycol, castor oil, tsubaki oil and liquid petrolatum.

Examples of the above-described "other ingredients" which are blended in the hair treatment agent include surfactant, emulsification stabilizer, thickener, microbicide, ultraviolet absorber, ultraviolet reflection scattering agent, antioxidant, perfume, vitamin, coloring material and various natural product extract ingredients, however, the present invention is not limited thereto.

The surfactant may be an anionic surfactant, a cationic surfactant or a nonionic surfactant.

Examples of the anionic surfactant include alkali salts (e.g., sodium salt, potassium salt, ammonium salt, triethanolamine salt) of higher fatty acids such as lauric acid, myristic acid, palmitic acid, stearic acid and oleic acid;
alkylsulfuric esters such as sodium lauryl sulfate and triethanolamine lauryl sulfate; and
alkylether sulfuric esters such as sodium polyoxyethylene laurylether sulfate and triethanolamine polyoxyethylene laurylether sulfate.

Examples of the nonionic surfactant include polyoxyethylene alkyl ethers such as polyoxyethylene laurylether and polyoxyethylene oleylether; and
alkanolamides such as coconut fatty acid diethanolamide and diethanolamide laurate.

Examples of the cationic surfactant include ammonium stearyltrimethyl chloride, ammonium cetyltrimethyl chloride and ammonium stearylbis(diethyl alcohol)hydroxyethyl chloride.

Examples of the emulsification stabilizer include cetanol, stearyl alcohol and behenyl alcohol.

Examples of the thickener include sodium alginate, methyl cellulose, carboxymethyl cellulose, polyvinyl alcohol and polyvinyl pyrrolidone.

Examples of the antiseptic include ethyl parahydroxybenzoate, butyl parahydroxybenzoate and benzalkonium chloride.

Examples of the ultraviolet absorber include para-aminobenzoic acid, ethylene glycol salicylate, octyl paramethoxycinnamate and hydroxybenzophenone.

Examples of the ultraviolet reflection scattering agent include red iron oxide, titanium oxide and zinc oxide.

Examples of the antioxidant include butyl hydroxy toluene, propyl gallate and butyl hydroxy anisole.

The perfume may be a perfume commonly used for general purposes and examples thereof include citrus, lavender and floral.

Examples of the vitamin include β-carotene, thiamine hydrochloride, folic acid, nicotinic acid amide and tocopherol.

Examples of the coloring material include azo pigments such as Food Red 2, Food Red 102 and Food Yellow 4;
triphenylmethane pigments such as Food Blue 1 and Food Green 3;
xanthene pigments such as Food Red 106 and Food Red 213;
quinoline pigments such as Food Yellow 203 and Food Yellow 204; and
natural pigments such as cochineal, shikonin, gardenia red and gardenia yellow.

Examples of the natural product extract ingredient include vegetable-base ingredients such as aloe extract, sea weed extract, sponge gourd extract and rosemary extract; and
animal-base ingredients such as sodium hyaluronate, placenta extract and mucopolysaccharide.

### Hair Care Composition and Use Thereof

The hair care composition of the present invention can be used as a hair washing composition such as rinse, hair treatment, hair conditioner, shampoo and pet shampoo, or as a hair styling composition such as setting lotion, hair cream, hair spray, hair tonic and foam.

The hair treatment agent is not particularly limited on the amount which can be blended in the hair care composition of the present invention as long as the amount blended is sufficient to attain the object of the present invention, and the hair treatment agent can be appropriately blended according to use, preparation form or the like.

As for other ingredients other than the above-described hair treatment agent, the hair washing composition and the hair-styling composition according to the present invention each may contain any ingredient as long as the ingredient can be generally used as cosmetic ingredients. Examples of the other ingredients include the same ingredients as described above for the hair treatment agent, such as base, surfactant, emulsification stabilizer, thickener, microbicide, ultraviolet absorber, ultraviolet reflection scattering agent, antioxidant, perfume, vitamin, coloring material and various natural product extract ingredients, however, the present invention is not limited thereto.

The hair washing composition and the hair-styling composition of the present invention each contains the above-described ascorbic acid derivative having excellent safety and low irritation to skin and being odorless and therefore, can be suitably used not only for human beings but also for pets.

This hair washing composition is mainly used at the time of washing hair in a bath or at the time of cleaning hair of a pet and examples of the use method include shampoo, rinse, hair pack, hair treatment, hair conditioner, pet shampoo and pet rinse.

The hair-styling composition is mainly used for finishing hair dressing, straightening curly hair or holding the hair set or wave after the hair dressing and examples of the use method include setting lotion, styling jell, hair cream, hair spray, hair foam, hair oil, hair tonic, hair liquid and hair solid.

Examples of the shampoo include a transparent shampoo where sodium alkylether sulfate, diethanolamide laurate, propylene glycol, antiseptic, dye, perfume, water and the like are blended in addition to the ascorbic acid derivative;
a creamy (pearl-like) shampoo where triethanolamine alkylsulfate, coconut fatty acid monoethanolamide, ethylene glycol monostearate, antiseptic, dye, perfume, water and the like are blended in addition to the ascorbic acid derivative;
an antidandruff shampoo where triethanolamine alkylsulfate, diethanolamide laurate, triethanolamine polyacrylate, Zpt, dye, perfume, water and the like are blended in addition to the ascorbic acid derivative;
a conditioning shampoo where sodium alkylether sulfate, coconut fatty acid diethanolamide, cation-denatured cellulose ether, antiseptic, dye, perfume, water and the like are blended in addition to the ascorbic acid derivative; and
a baby shampoo where imidazoline-type amphoteric surfactant, sodium alkylether sulfate, polyoxyethylene sorbitan fatty acid ester, antiseptic, dye, perfume, water and the like are blended in addition to the ascorbic acid derivative.

Examples of the rinse include a cream rinse where ammonium stearyltrimethyl chloride, cetanol, behenyl alcohol, 1,3-propylene glycol, butyl parahydroxy benzoate, purified water and the like are blended in addition to the ascorbic acid derivative as the essential ingredient;
an oil rinse where ammonium stearyltrimethyl chloride, polyoxyethylene cetyl ether, polyoxyethylene lanoline ether, propylene glycol, citric acid, sodium citrate, butyl parahydroxybenzoate, methyl para-hydroxybenzoate, perfume, water and the like are blended in addition to the ascorbic acid derivative as the essential ingredient; and
a conditioning rinse where denatured starch, polyoxyethylene cholesterol, glycerol monostearate, silicone, perfume, water and the like are blended in addition to the ascorbic acid derivative as the essential ingredient.

Examples of the styling jell include a styling jell containing ingredients of carboxymethyl cellulose, triethanolamine, glycerol, polyvinyl pyrrolidone, ethyl parahydroxybenzoate, 1,3-butylene glycol, purified water and the like, in addition to the ascorbic acid derivative as the essential ingredient.

### Effects of the Invention

The hair treatment agent of the present invention can easily and simply scavenge hydroxy radicals which are generated under the action of ultraviolet rays or the like and harmful to hair.

The ascorbic acid derivative contained in the hair treatment agent has sufficiently high storage stability required in view of the production of preparations.

Furthermore, the hair treatment agent of the present invention comprises an ascorbic acid derivative originated from a natural ingredient and therefore, this is not only excellent in the safety to skin, low in the irritation, harmless even when attached to skin on use of the hair treatment agent, and safe and harmless even if penetrated into skin, but also advantageous in that the synthesis of collagen in the skin tissue is stimulated and the activity of protecting the skin tissue from external factors such as ultraviolet ray is strengthened. Accordingly, the hair treatment agent of the present invention can be suitably used for protecting hair of human beings. In addition, since the ascorbic acid derivative as the effective ingredient of the hair treatment agent of the present invention is odorless, the hair treatment agent of the present invention can also be suitably used for hair of pets which are highly sensitive to odors.

Also, the hair treatment agent of the present invention can be blended in a hair care composition such as hair tonic, rinse, hair conditioner, shampoo and hair cream, and can provide effective uses simultaneously having additional functions such as washing and nutrient supply.

### Examples

Examples and Formulation Examples of the hair treatment agent and the hair care composition according to the present invention are shown below, however, the present invention is not limited to these Examples and Formulation

### Examples.

In the following Examples and Formulation Examples, unless otherwise indicated, the concentration indicates a concentration in the unit of % by mass. Furthermore, in the following Examples and Formulation Examples, ascorbyl 2-phosphate sodium salt is sometimes simply referred to as "APS", ascorbyl 2-phosphate magnesium salt as "APM" and ascorbyl 2-glucoside as "AG".

Using hair treatment agents according to the present invention, the dechlorinating ability thereof, the ability of preventing "decolorization of melanin pigment caused by chlorine", the ability of preventing "hair damage caused by chlorine", the hydroxy radical-scavenging ability (radical-scavenging ability) and the ability of preventing "hair damage caused by sunlight" were determined below.

The measuring methods and the results obtained are shown below.

### (Test Example 1)

### Measurement of Dechlorinating Ability of Ascorbyl 2-phosphate Sodium Salt (APS) and Ascorbyl 2-glucoside (AG)

To chlorine water having a previously determined free chlorine concentration, APS or AG was added and the amount of free chlorine decreased was measured. The free chlorine amount was measured by iodometric titiration.

To each of three Erlenmeyer flasks with a stopper, 10 ml of chlorine water having a known free chlorine concentration was added and thereto, APS or AG in an equimolar amount, 1.5 times molar amount or 2 times molar amount to the free chlorine in the solution was added and stirred. After 5 minutes, 10 minutes, 15 minutes and 20 minutes, the amount of free chlorine was titrated.

In the titration, 2 ml of each reaction solution was charged into another Erlenmeyer flask with a stopper, 50 ml of distilled water was added thereto, 1.0 g of potassium iodide and 1 ml of a 0.2% starch solution were further added and after stirring, a 0.1 N sodium thiosulfate standard solution was added dropwise. The point where purple color was faded out was designated as a final point. From the amount of sodium thiosulfate added dropwise at this time, the amount of free chlorine was calculated.

The results obtained are shown in Tables 1 and 2.

**Table 1**

| Dechlorinating Ability of Hair Treatment Agent | | | | | |
|---|---|---|---|---|---|
| (ascorbyl 2-phosphate sodium salt) | | | | | |
| Molar Ratio, Molar Number of APS : Molar Number of Free Chlorine | Initial Free Chlorine | After 5 Minutes, % | After 10 Minutes, % | After 15 Minutes, % | After 20 Minutes, % |
| 1.0 : 1.0 | 0.43 | 0.02 | 0.012 | 0.008 | 0 |
| 1.5 : 1.0 | 0.43 | 0.005 | 0 | 0 | 0 |
| 2.0 : 1.0 | 0.43 | 0 | 0 | 0 | 0 |

| | | | | | |
|---|---|---|---|---|---|
| APS: ascorbyl 2-phosphate sodium salt | | | | | |

**Table 2**

| Dechlorinating Ability of Hair Treatment Agent | | | | | |
|---|---|---|---|---|---|
| (ascorbyl 2-glucoside) | | | | | |
| Molar Ratio, Molar Number of AG : Molar Number of Free Chlorine | Initial Free Chlorine | After 5 Minutes, % | After 10 Minutes, % | After 15 Minutes, % | After 20 Minutes, % |
| 1.0 : 1.0 | 0.43 | 0.033 | 0.02 | 0.01 | 0 |
| 1.5 : 1.0 | 0.43 | 0.008 | 0 | 0 | 0 |
| 2.0 : 1.0 | 0.43 | 0 | 0 | 0 | 0 |

| | | | | | |
|---|---|---|---|---|---|
| AG: ascorbyl 2-glucoside | | | | | |

### (Test Example 2)

### Measurement of Ability of Preventing "Decolorization of Melanin Pigment caused by Chlorine" when Ascorbyl 2-phosphate Sodium Salt (APS) or Ascorbyl 2-glucoside (AG) is Added

A melanin pigment (*sepia officinailis*) was added to a 2 N aqueous sodium hydroxide solution and dissolved under heating at 80°C for 2 hours to adjust the melanin pigment content in the solution to 50 ppm. This melanin pigment solution was divided into 5 vessels and 5 kinds in total of sample solutions were prepared, namely, solutions adjusted to an APS concentration of 10 mM and 50 mM, solutions adjusted to an AG concentration of 10 mM and 50 mM, and a control having added thereto neither APS nor AG. Then, 4 ml of each solution was sampled in a sampling bottle and 0.5 ml of commercially available chlorine water (free chlorine concentration: about 0.43%) was added to each sampling bottle. As a blank, a melanin pigment solution was used. The prevention of melanin decolorization was evaluated by measuring the absorbance (abs) at 400 nm using a spectrophotometer when 3 hours (3H) and 6 hours (6H) were passed after the addition of chlorine water. The results obtained are shown in Table 3.

**Table 3**

| Ability of Preventing "Decolorization of Melanin Pigment" | | | | | | |
|---|---|---|---|---|---|---|
| | Concent- ration of Additive (mM) | Before Addition | 3 Hours After Addition | 6 Hours After Addition | Residual Ratio, 3H | Residual Ratio, 6H |
| | | abs | abs | abs | | |
| APS | 10 | 0.577 | 0.449 | 0.453 | 0.778 | 0.785 |
| | 50 | 0.592 | 0.545 | 0.505 | 0.921 | 0.853 |
| AG | 10 | 0.575 | 0.458 | 0.447 | 0.797 | 0.777 |
| | 50 | 0.569 | 0.553 | 0.540 | 0.972 | 0.949 |
| Control | - | 0.583 | 0.257 | 0.205 | 0.441 | 0.352 |
| Blank | - | 0.583 | - | 0.544 | - | 0.933 |

| | | | | | | |
|---|---|---|---|---|---|---|
| APS: ascorbyl 2-phosphate sodium salt AG: ascorbyl 2-glucoside (AG) Control: no addition of APS and AG Blank: melanin pigment solution | | | | | | |

### (Test Example 3)

### Measurement of Ability of Preventing "Hair Damage caused by Chlorine" when Ascorbyl 2-phosphate Sodium Salt (APS) or Ascorbyl 2-glucoside (AG) is Added

120 ml of an aqueous 10 mM APS solution, 120 ml of an aqueous 10 mM AG solution and 120 ml of purified water each was poured into two bottles with a screw opening. In the aqueous 10 mM APS solution, the aqueous 10 mM AG solution or the purified water, hair of an adult woman and hair of a one-year-old boy, which were previously subjected to the measurement of color (L*a*b* color specification) by a spectrocolorimeter (Macbeth Gretag APM50), each was immersed in an amount of 70 to 80 mg while stirring. To each solution in which the hair was immersed, 15 ml of a commercially available chlorine water (concentration of free chlorine: about 0.43%) was added. After stirring for one hour, the hair was filtered, washed with water and air-dried. The resulting hairs were measured on the color (L*a*b* color specification) and from the values obtained, the color difference ΔE value was calculated.

The results are shown in Table 4.

**Table 4**

| ΔE Value of Hair Treated With Chlorine, | | |
|---|---|---|
| | ΔE | |
| | Adult Woman | One-Year-Old Boy |
| Ascorbyl 2-phosphate sodium salt(APS) | 0.4 | 0.5 |
| Ascorbyl 2-glucoside (AG) | - | 1.1 |
| Purified water (control) | 5.3 | 14.4 |

### (Test Example 4)

### Measurement of Hydroxy Radical-Scavenging Ability of Ascorbyl 2-phosphate Sodium Salt (APS), Ascorbyl 2-phosphate Magnesium Salt (APM) and Ascorbyl 2-glucoside (AG)

Using "ESR-spin trap method", the radical scavenging in percentage of each of ascorbyl 2-phosphate sodium salt (APS), ascorbyl 2-phosphate magnesium salt (APM) and ascorbyl 2-glucoside (AG) was measured. The radicals were chemically generated.

10 µl of 1M HEPES buffer solution (pH: 7.2), 10 µl of 1 mM diethylenetriaminepentaacetic acid, each of 20 µl of APS, APM or AG solution, 100 µl of purified water, 40 µl of 40 mM 2,2'-azobis(2-methylpropionamidine)dihydrochloride and 20 µl of 0.5 M 5,5-dimethyl-1-pyrroline-N-oxide were added and mixed. The obtained solutions were sequentially enclosed into flat cells for ESR and the cells were placed in an ESR apparatus ("JES-RE1X", manufactured by JEOL Ltd.) and subjected to the measurement of hydroxy radical-scavenging ability. The hydroxy radical-scavenging ability is shown by S/M value (area value of spectrum/area value of manganese marker) and the scavenging ratio is shown by percentage (%).

The results are shown in Table 5.

**Table 5**

| Measurement of Radical Scavenging Ability of Hair Treatment Agent (Ascorbic Acid Derivatives), | | | | | | |
|---|---|---|---|---|---|---|
| Concentration (mM) | Ascorbyl 2-phosphate Sodium Salt (APS) | | Ascorbyl 2-phosphate Magnesium Salt (APM) | | Ascorbyl 2-glucoside | |
| | S/M | % | S/M | % | S/M | % |
| 0 | 0.65 | 100.0 | 0.65 | 100.0 | 0.65 | 100.0 |
| 0.01 | 0.67 | 103.1 | 0.58 | 89.2 | 0.64 | 98.5 |
| 0.03 | 0.68 | 104.6 | 0.60 | 92.3 | 0.58 | 89.2 |
| 0.1 | 0.51 | 78.5 | 0.53 | 81.5 | 0.64 | 98.5 |
| 0.3 | 0.35 | 53.8 | 0.42 | 64.6 | 0.59 | 90.8 |
| 1 | 0.11 | 16.9 | 0.12 | 18.5 | 0.40 | 61.5 |

### (Test Example 5)

### Measurement of Ability of Preventing "Hair Damage caused by Sunlight" When Ascorbyl 2-phosphate Sodium Salt (APS) or Ascorbyl 2-glucoside (AG) is Added

An APS solution, an AG solution, which were each adjusted to a pH of 7.2 and a concentration of 10 mM or 100 mM, and purified water (control) each was charged into a polyethylene bag and in respective bags, from 70 to 80 g of hair previously measured on the color (L*a*b* color specification) by a spectrocolorimeter (Macbeth Gretag APM50) was enclosed. After the immersion for about 2 hours, light was irradiated thereon using a weather meter (high energy xenon weather meter, manufactured by Suga Shikenki K.K.). The irradiation time was 24 hours and the integrated light intensity was about 10,000 kj/m². The hair after the irradiation was washed with purified water, air-dried and then subjected to the measurement of color (L*a*b* color specification) and from the values obtained, the color difference ΔE value was calculated.

The results are shown in Table 6.

**Table 6**

| ΔE Value of Weather Meter Treated-hair | | |
|---|---|---|
| | Concentration | ΔE Value |
| | (mM) | |
| Ascorbyl 2-phosphate Sodium Salt (APS) | 10 | 14.36 |
| | 100 | 4.54 |
| Ascorbyl 2-glucoside (AG) | 10 | 10.72 |
| | 100 | 6.01 |
| Purified Water (Control) | - | 14.14 |

### (Blend Example 1)

### Styling Jell

| | (Ingredient Blended) | (Amount Blended) |
|---|---|---|
| A: | Ascorbyl 2-phosphate sodium salt | 5.0% |
| | Carboxymethyl cellulose | 1.0% |
| | Purified water | 15.0% |
| B: | Triethanolamine | 0.5% |
| | Purified water | 4.5% |
| C: | Glycerin | 30.0% |
| | Polyvinyl pyrrolidone | 5.0% |
| | Purified water | 5.0% |
| D: | Ethyl parahydroxybenzoate | 0.1% |
| | 1,3-Butylene glycol | 10.0% |
| | Purified water | an amount to make 100% in total of all ingredients |

### <Preparation Method>

Respective ingredients constituting D were heated at 80°C and uniformly dissolved and the resulting solution was cooled to an ordinary temperature to prepare Ingredient D. Similarly, respective ingredients of each of A, B and C were mixed and dissolved at an ordinary temperature to prepare Ingredient A, Ingredient B and Ingredient C. In the thus-prepared Ingredient A, Ingredients B to D prepared similarly were added in the order of B, C and D while stirring. The obtained solution was further stirred to be uniform and left standing, thereby obtaining a styling jell.

### (Blend Example 2)

### Cream Rinse

| | (Ingredient Blended) | (Amount Blended) |
|---|---|---|
| A: | Ascorbyl 2-phosphate sodium salt | 5.0% |
| | Stearyltrimethyl-ammonium | 4.0% |
| | chloride | |
| | Cetanol | 2.5% |
| | Behenyl alcohol | 2.0% |
| | 1,3-Propylene glycol | 5.0% |
| | Butyl parahydroxybenzoate | 0.1% |
| B: | Purified water | an amount making 100% in total of all ingredients |

### <Preparation Method>

Respective ingredients in A above were heated at 80°C and dissolved to prepare Ingredient A. To the-thus obtained Ingredient A, Ingredient B previously heated at 80°C was added while stirring and emulsified. The stirring was further continued and when the solution temperature reached from 35 to 40°C, the stirring was stopped and the solution was left standing, as a result, a cream rinse was obtained.

## Claims

1. A use of a composition of a hair treatment agent to prevent damage of the hair caused by hydroxyl radicals, the composition comprising, as an effective ingredient, an ascorbic acid derivative which is a compound represented by formula (1) or a salt thereof: 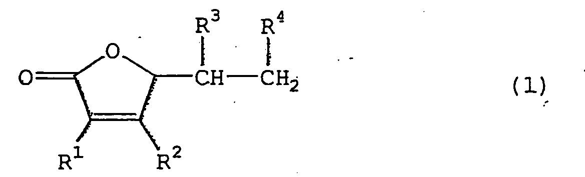 (wherein R¹, R², R³ and R⁴ each independently represents a hydroxyl group, an ester bound-containing group derived from a hydroxyl group and an organic group or an inorganic group; or R¹ and R² or R³ and R⁴ may form together a divalent group derived from two adjacent hydroxyl groups and aldehyde or ketone, provided that R¹ and R² are not a hydroxyl group at the same time).

2. The use of a composition of a hair treatment agent to prevent damage of the hair caused by hydroxyl radicals as claimed in claim 1, wherein said ascorbic acid derivative has a dechlorinating ability and a hydroxyl radical-scavenging ability.

3. The use of a composition of a hair treatment agent to prevent damage of the hair caused by hydroxyl radicals as claimed in claim 1 or 2, wherein said ester bond-containing group is an acyloxy group or a group selected from the group consisting of a sulfuric acid group, a phosphoric acid group, a pyrophosphoric acid group, a triphosphoric acid group and polyphosphoric acid group.

4. The use of a composition of a hair treatment agent to prevent damage of the hair caused by hydroxyl radicals as claimed in any one of claims 1 to 3, wherein said ascorbic acid derivative is ascorbyl 2-phosphate ester or a salt thereof.

5. The use of a composition of a hair treatment agent to prevent damage of the hair caused by hydroxyl radicals as claimed in any one of claims 1 to 4, wherein the hair treatment agent is in a liquid form and the ascorbic acid derivative content in the hair treatment agent is from 0.01 to 50% by mass.

6. The use according to claim 1, wherein the composition is a hair care composition for hair-styling.

7. The use according to claim 1, wherein the composition is a hair care composition for pets.

8. The use according to claim 1, wherein the composition is a hair care composition in the form of a shampoo, rinse, hair conditioner, hair tonic or styling gel.

## Patentansprüche

1. Verwendung einer Zusammensetzung eines Haarbehandlungsmittels zur Verhinderung der durch Hydroxyradikale verursachten Beschädigung von Haar, wobei die Zusammensetzung als wirksamen Bestandteil ein Ascorbinsäurederivat enthält, das eine Verbindung der Formel (1) oder ein Salz davon ist: 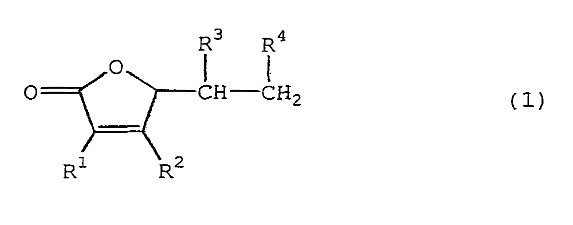 (worin R¹, R², R³ und R⁴ jeweils unabhängig voneinander eine Hydroxygruppe, eine von einer Hydroxygruppe und einer organischen Gruppe oder anorganischen Gruppe abgeleitete Gruppe mit einer Esterbindung darstellt; oder R¹ und R² oder R³ und R⁴ zusammen eine zweiwertige Gruppe bilden können, die von 2 benachbarten Hydroxygruppen und Aldehyd oder Keton abgeleitet ist, mit der Maßgabe, dass R¹ und R² nicht gleichzeitig eine Hydroxygruppe sind).

2. Verwendung einer Zusammensetzung eines Haarbehandlungsmittels zur Verhinderung der durch Hydroxyradikale verursachten Beschädigung von Haar nach Anspruch 1, wobei das Ascorbinsäurederivat die Fähigkeit zur Chlorentfernung und zum Abfangen von Hydroxyradikalen hat.

3. Verwendung einer Zusammensetzung eines Haarbehandlungsmittels zur Verhinderung der durch Hydroxyradikale verursachten Beschädigung von Haar nach Anspruch 1 oder 2, wobei die Esterbindung enthaltende Gruppe eine Acyloxygruppe oder eine Gruppe ist, die aus der Gruppe ausgewählt ist, die aus einer Schwefelsäuregruppe, einer Phosphorsäuregruppe, einer Pyrophosphorsäuregruppe, einer Triphosphorsäuregruppe und einer Polyphosphorsäuregruppe besteht.

4. Verwendung einer Zusammensetzung eines Haarbehandlungsmittels zur Verhinderung der durch Hydroxyradikale verursachten Beschädigung von Haar nach einem der Ansprüche 1 bis 3, wobei das Ascorbinsäurederivat Ascorbyl-2-phosphatester oder ein Salz davon ist.

5. Verwendung einer Zusammensetzung eines Haarbehandlungsmittels zur Verhinderung der durch Hydroxyradikalen verursachten Beschädigung von Haar nach einem der Ansprüche 1 bis 4, wobei das Haarbehandlungsmittel in flüssiger Form vorliegt und der Ascorbinsäurederivatgehalt in dem Haarbehandlungsmittel 0,01 bis 50 Massen-% ist.

6. Verwendung nach Anspruch 1, wobei die Zusammensetzung eine Haarpflegezusammensetzung zum Frisieren ist.

7. Verwendung nach Anspruch 1, wobei die Zusammensetzung eine Haarpflegezusammensetzung für Haustiere ist.

8. Verwendung nach Anspruch 1, wobei die Zusammensetzung eine Haarpflegezusammensetzung in der Form eines Schampoos, einer Spülung, einer Haarkur, eines Haartonikums oder eines Frisiergels ist.

## Revendications

1. Utilisation d'une composition d'un agent de traitement capillaire destinée à éviter la détérioration des cheveux occasionnée par des radicaux hydroxyle, la composition comprenant, comme ingrédient effectif, un dérivé d'acide ascorbique qui est un composé représenté par la formule (1) ou un sel de celui-ci : 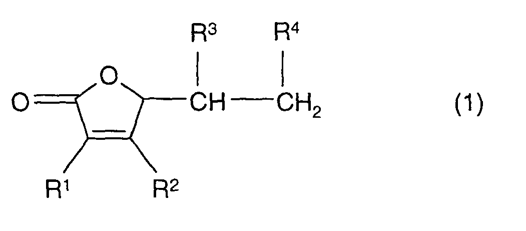 (dans laquelle R¹, R², R³ et R⁴ représentent chacun indépendamment un groupe hydroxyle, un groupe contenant une liaison ester dérivé d'un groupe hydroxyle et d'un groupe organique ou d'un groupe inorganique ; ou R¹ et R² ou R³ et R⁴ peuvent former ensemble un groupe divalent dérivé de deux groupes hydroxyle adjacents et d'un aldéhyde ou d'une cétone, à condition que R¹ et R² ne soient pas simultanément un groupe hydroxyle).

2. Utilisation d'une composition d'un agent de traitement capillaire destinée à éviter la détérioration des cheveux occasionnée par des radicaux hydroxyle selon la revendication 1, dans laquelle ledit dérivé d'acide ascorbique présente une aptitude à la déchloration et une aptitude à la fixation de radical hydroxyle.

3. Utilisation d'une composition d'un agent de traitement capillaire destinée à éviter la détérioration des cheveux occasionnée par des radicaux hydroxyle selon la revendication 1 ou 2, dans laquelle ledit groupe contenant une liaison ester est un groupe acyloxy ou un groupe choisi parmi un groupe d'acide sulfurique, un groupe d'acide phosphorique, un groupe d'acide pyrophosphorique, un groupe d'acide triphosphorique et un groupe d'acide polyphosphorique.

4. Utilisation d'une composition d'un agent de traitement capillaire destinée à éviter la détérioration des cheveux occasionnée par des radicaux hydroxyle selon l'une quelconque des revendications 1 à 3, dans laquelle ledit dérivé d'acide ascorbique est un ester de 2-phosphate d'ascorbyle ou un sel de celui-ci.

5. Utilisation d'une composition d'un agent de traitement capillaire destinée à éviter la détérioration des cheveux occasionnée par des radicaux hydroxyle selon l'une quelconque des revendications 1 à 4, dans laquelle l'agent de traitement capillaire est dans une forme liquide et la teneur en dérivé d'acide ascorbique dans l'agent de traitement capillaire est de 0,01 à 50 % en masse.

6. Utilisation selon la revendication 1, dans laquelle la composition est une composition de soin capillaire pour le coiffage.

7. Utilisation selon la revendication 1, dans laquelle la composition est une composition de soin capillaire destinée aux animaux domestiques.

8. Utilisation selon la revendication 1, dans laquelle la composition est une composition de soin capillaire dans la forme d'un shampoing, d'un rinçage, d'un après-shampoing, d'un tonique capillaire ou d'un gel de coiffage.
